# EUROPEAN PATENT SPECIFICATION

(11) **EP 0 421 367 B1**
(45) Date of publication and mention of the grant of the patent: **22.04.1998**
(21) Application number: 90118903.5
(22) Date of filing: 03.10.1990
(51) Int. Cl.: C07K 7/06, A61K 38/00

(54) **Anticoagulant peptides**
Antikoagulierende Peptide
Peptides anticoagulants

(30) Priority: 03.10.1989 US 416336
(43) Date of publication of application: 10.04.1991
(73) Proprietor: MERRELL PHARMACEUTICALS INC., Cincinnati, Ohio 45215-6300 (US)
(72) Inventor: Krstenansky, John L., Cincinnati, OH 45208 (US)
(74) Representative: VOSSIUS & PARTNER

(56) References cited:
- EP-A- 0 276 014
- EP-A- 0 333 356

## Description

This invention relates to novel anticoagulant peptides and as such are also valuable reagents to the development of anticoagulants.

Anticoagulants are useful therapeutic agents in the pharmacological treatment of, for example, acute deep venous thrombosis, pulmonary embolism, acute arterial embolization of the extremities, myocardial infarction, and disseminated intravascular coagulation. Prophylactic administration of anticoagulants is believed to prevent a recurrence of embolism in patients with rheumatic or arteriosclerotic heart disease and to prevent certain thromboembolic complications of surgery. Administration of anticoagulants has also been indicated in the treatment of coronary artery and cerebrovascular disease. Arterial thrombosis, particularly in arteries supplying the heart muscle and brain, is a leading cause of death.

Hirudin is a 65 residue polypeptide isolated from the salivary glands of leeches. It is an anticoagulant agent, which is a thrombin specific inhibitor. Although quite potent, clinical use of hirudin isolated from leech extracts seems unlikely because of its limited quantity, expense, and allergic reactions which commonly follow administration of any foreign protein of this size.

Originally, applicants discovered a specific region of hirudin that is responsible, at least in part, for its anticoagulant activity . The peptide region (amino acid 55 to 65 of Hirudin) was chemically synthesized and shown to bind the recognition site of thrombin; the recognition site being spatially distinct from the the enzymatic cleavage site. Binding of synthetic peptides is also shown to competitively prevent binding of fibrinogen to the recognition site of thrombin, an important prerequisite to fibrin production and clot formation, and are thereby of potential medical value as anticoagulants; see EP-A-0 276 014 and EP-A-0 333 356.

Applicants have further prepared derivatives of this peptide containing single amino acid deletions of the basic sequence. Specifically, this series of amino acid deletions, taken in total, define the extent of sequence dependency of the peptide, positionally and compositionally, and provide the basis for extended rational drug design. Many of the peptide analogs of this type have attributes of the parent peptide and therefore may also serve as a scientifically interesting and therapeutically significant adjunct to anticoagulant therapy. Moreover, the amino acid analogs may in themselves contain enhanced potency and extended duration of action.

A peptide derivative selected from 
Suc-Tyr-Pro-Ile-Pro-Glu-Glu-Ala-Cha-D-Glu-OH;
H-Gly-Asp-Phe-Glu-Glu-Ile-Glu-Glu-Tyr-Leu-Gln-OH;
Suc-Tyr-Glu-Pro-Tiq-Glu-Glu-Ala-Cha-D-Glu-OH;
Suc-Tyr-Glu-Pro-Ile-Pro-Glu-Glu-Ala-Gln-NH2;
Suc-Tyr-Glu-Pro-Ile-Pro-Glu-Glu-Ala-Asn-OH;
Mal-Tyr-Pro-Ile-Pro-Glu-Glu-Ala-Cha-D-Glu-OH;
Suc-Nap-Pro-Ile-Pro-Glu-Glu-Ala-Cha-D-Glu-OH;
Suc-Tyr-Glu-Ile-Pro-Glu-Glu-Ala-Cha-D-Glu-OH;
Suc-Tyr-Glu-Pro-Pro-Glu-Glu-Ala-Cha-D-Glu-OH;
Suc-Tyr-Glu-Pro-Ile-Glu-Glu-Ala-Cha-D-Glu-OH;
Suc-Tyr-Glu-Pro-Ile-Pro-Glu-Glu-Ala-D-Glu-OH;
Suc-Tyr-Glu-Pro-Ile-Pro-Glu-Glu-Ala-Gln-OH;
Suc-Phe-Glu-Pro-Ile-Pro-Glu-Glu-Ala-Gln-NH2;
Suc-Glu-Pro-Ile-Pro-Glu-Glu-Ala-Cha-D-Glu-OH;
 or pharmaceutical acceptable salt thereof is an useful anticoagulant agent.

The following common abbreviations of; (1) amino acids and their three letter code, (2) modified and unusual amino acids, and (3) terminal amino and carboxy substituents used throughout this specification:

### (1): THE AMINO ACIDS AND THEIR THREE LETTER CODE

| L-AMINO ACIDS | D-AMINO ACIDS |
|---|---|
| Ala (or A) - alanine | D-Ala (or a) - D-alanine |
| Arg (or R) - arginine | D-Arg (or r) - D-arginine |
| Asn (or N) asparagine | D-Asn (or n) - D-asparagine |
| Asp (or D) - aspartic acid | D-Asp (or d) - D-aspartic acid |
| | |
| Cys (or C) - cysteine | D-Cys (or c) - D-cysteine |
| Gly (or G) - glycine | |
| Glu (or E) - glutamic acid | D-Glu (or e) - D-glutamic acid |
| | |
| Val (or V) - valine | D-Val (or v) - D-valine |
| Gln (or Q) - glutamine | D-Gln (or q) - D-glutamine |
| His (or H) - histidine | D-His (or h) - D-histidine |
| Ile (or I) - isoleucine | D-Ile (or i) - D-isoleucine |
| Leu (or L) - leucine | D-Leu (or l) - D-leucine |
| Lys (or K) - lysine | D-Lys (or k) - D-lysine |
| Phe (or F) - phenylalanine | D-Phe (or f) - D-phenylalanine |
| | |
| Met (or M) - methionine | D-Met (or m) - D-methionine |
| Pro (or P) - proline | D-Pro (or p) - D-proline |
| Ser (or S) - serine | D-Ser (or s) - D-serine |
| Thr (or T) - threonine | D-Thr (or t) - D-threonine |
| Trp (or W) - tryptophan | D-Trp (or w) - D-tryptophan |
| Tyr (or Y) - tyrosine | D-Tyr (or y) - D-tyrosine |

### (2): MODIFIED AND UNUSUAL AMINO ACIDS

Aba - α-amino-n-butyric acid
pClPhe - para-chloro-phenylalanine
Cha - cyclohexylalanine
Chg - cyclohexylglycine
Hyp - hydroxyproline
I-Tyr - 3-iodotyrosine, 5-iodotyrosine, 3,5-diiodotyrosine,
γMeGlu - D-glutamic acid gamma methyl ester
NMePhe - N-methyl phenylalanine
NMePgl - N-methyl phenylglycine
Npa - β-(naphthyl)alanine
3,4-dehydroPro - 3,4-dehydroproline
pNO2Phe - para-nitro-phenylalanine
Nle - norleucine
Orn - ornithine
Pip - pipecolate
Pba - p-aminophenyl butyric acid
pSubPhe - para substituted phenylalanine
Pgl - phenylglycine
Sar - sarcosine (N-methylglycine)
SubPhe - ortho, meta, or para, mono- or di- substituted phenylalanine
Tha - β-(2-thienyl)-alanine
Tiq - Tetrahydroisoquinoline 3-carboxylate

### AMINO AND CARBOXY TERMINAL ACID SUSTITUENTS

Ac - acetyl
Azt - azetidine-2-carboxylate
Cin - cinnamoyl
3,4-dihydroCin - 3,4-dihydrocinnamoyl
Glt - glutaryl
Mal - maleyl
Oac - 8-aminooctanoic acid
Oct - n-octyl
Suc - succinyl
Glt - glutaryl
Tfa - trifluoroacetyl
# - C-terminal amide

The following designates the known naturally occurring amino acid sequence variations of Hirudin:

AMINO ACID SEQUENCE VARIATIONS OF HIRUDIN

1 2 3 4 5 6 7 8 9 10 11 12 13 14
Val Val Tyr Thr Asp Cys Thr Glu Ser Gly Gln Asn Leu Cys
Ile Thr

15 16 17 18 19 20 21 22 23 24 25 26 27 28
Leu Cys Glu Gly Ser Asn Val Cys Gly Gln Gly Asn Lys Cys
Lys

29 30 31 32 33 34 35 36 37 38 39 40 41 42
Ile Leu Gly Ser Asp Gly Glu Lys Asn Gln Cys Val Thr Gly
Asn Lys Gly
Gln Asp

43 44 45 46 47 48 49 50 51 52 53 54 55 56
Glu Gly Thr Pro Lys Pro Glx Ser His Asn Asp Gly Asp Phe
Asn Glu

57 58 59 60 61 62 63 64 65
Glu Glu ILe Pro Glu Glu Tyr Leu Gln
Pro Asp Asp Glu
(Ala63 Tyr64 Leu/Asp64 Glu65)

The naturally occurring amino acids, with the exception of glycine, contain a chiral carbon atom. Unless otherwise specifically indicated, the optically active amino acids, referred to herein, are of the L-configuration. For example, any of the amino acids can be of the D- or L-configuration. As is customary, the structure of peptides written out herein is such that the amino terminal end is on the left side of the chain and the carboxy terminal end is on the right side of the chain.

An alkyl group and the alkyl portion of an alkoxy group are taken to include straight, branched, or cyclic alkyl groups, for example, methyl, ethyl, propyl, isopropyl, butyl, isobutyl, tert-butyl, pentyl, isopentyl, sec-pentyl, cyclopentyl, hexyl, isohexyl, cyclohexyl and cyclopentylmethyl, heptyl, octyl(Oct), 8-aminooctanoic acid(Oac). An acyl group of from 2 to 10 carbon atoms is taken to include straight, branched, cyclic, saturated and unsaturated acyl groups having 1 or 2 carbonyl moieties per group, for example, acetyl(Ac), azetidine-2-carboxylate(Azt), benzoyl succinyl, cinnamoyl(Cin), 3,4-dihydrocinnamoyl (3,4-dihydroCin), maleyl(Mal), and glutaryl(Glt). Both alkyl and acyl substituents are taken to include those groups with halogen substituents, were a halogen group is a fluoro, chloro, bromo or iodo, for example, trifluoroacetyl(Tfa).

The term "any amino acid" as used herein does not purport to include any carboxylic acid having an amino substituent, but rather is used as it is commonly used by those skilled in the art of polypeptide derivatives and includes the naturally occurring amino acids as well as other "non-protein" α-amino acids commonly utilized by those in the peptide chemistry arts when preparing synthetic analogs of naturally occurring peptides. The naturally occurring amino acids are the "L-amino acids" glycine, alanine, valine, leucine,isoleucine, serine, methionine, threonine, phenylalanine, tyrosine, tryptophan, cysteine, proline, histidine, aspartic acid, asparagine, glutamic acid, glutamine, arginine, ornithine, and lysine. Also included as "any amino acid" would be the D-isomers ("D-amino acids") of the naturally occurring L-amino acids; D-alanine, D-valine, D-leucine, D-isoleucine, D-serine, D-methionine, D-threonine, D-phenylalanine, D-tyrosine, D-tryptophan, D-cysteine, D-proline, D-histidine, D-aspartic acid, D-asparagine, D-glutamic acid, D-glutamine, D-arginine. Also included are "non-protein" α-amino acids, examples are norleucine, norvaline, alloisoleucine, homoarginine, thiaproline, dehydroproline, hydroxyproline (Hyp), homoserine, cyclohexylglycine (Chg), α-amino-n-butyric acid (Aba), cyclohexylalanine (Cha), aminophenylbutyric acid (Pba), phenylalanines mono or disubstituted at the ortho, meta, or para position, such as para substituted phenylalanine (pSubPhe), such as para-chlorophenylalanine and para-nitrophenylalanine (pNO₂Phe), or wherein positions of the phenyl moiety are substituted with one or two of the following, a (C₁-C₄) alkyl, (C₁-C₄) alkoxy, halogen, or nitro groups or substituted with a methylenedioxy group, β-2- and 3-thienyl-alanine, β-2- and 3-furanylalanine, β-2-, 3-,and 4-pyridylalanine, β-(benzothienyl-2- and 3-yl)alanine, β-(1-and 2-naphthyl)alanine(Npa), O-alkylated derivates of serine, threonine, or tyrosine, methyl esters of glutamic and aspartic acids, S-alkylated cysteine, the O-sulfate ester of tyrosine, and halogenated tyrosines such as 3-iodotyrosine, 5-iodotyrosine, 3,5-diiodotyrosine.

By the expression "sequences of hirudin or its natural variants" applicants intend that the amino acid sequences found for hirudin in nature apply.

The term "portions thereof" of Hirudin and its variants is meant to include a consecutive region of 4 amino acids derived from the sequence of hirudin or its variants.

The term "lipophilic amino acid" includes Tyr, Phe, Leu Met, Nle, Ile, Val, and Pro. Further, the term "imino acids" is meant to include all N-alkyl amino acids. Examples of imino acids would be N-methyl phenylalanine (NMePhe), N-methyl phenylglycine (NMePgl), (3,4-dehydroproline (3,4-dehydroPro), p-aminophenyl butyric acid (Pba), sarcosine (Sar), proline (Pro), and pipecolate (Pip). The expression "a peptide containing from 1-11 residues of any amino acid" is meant to reflect that addition of amino acids to either the amino or carboxy terminal end of the core amino acids encompass the core structure with its intrinsic activity.

The polypeptides of the present invention can form pharmaceutically acceptable salts with any non-toxic, organic or inorganic acid. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono, di and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic, and sulfonic acids such as methane sulfonic acid and 2-hydroxyethane sulfonic acid. Salts of the carboxy terminal amino acid moiety include the non-toxic carboxylic acid salts formed with any suitable inorganic or organic bases. Illustratively, these salts include those of alkali metals, as for example, sodium and potassium; alkaline earth metals, such as calcium and magnesium; light metals of Group IIIA including aluminum; and organic primary, secondary and tertiary amines, as for example, trialkylamines, including triethylamine, procaine, dibenzylamine, 1-etheneamine, N,N'-dibenzylethylenediamine, dihydroabietylamine, N-(lower)alkylpiperidine, and any other suitable amine.

### Synthesis:

The proteins of this invention can be prepared by a variety of procedures readily known to those skilled in the art. Such procedures include solution phase peptide synthesis, the solid phase sequential and block synthesis, gene cloning and combinations of these techniques. The solid phase sequential procedure can be performed using established automated methods such as by use of an automated peptide sythesizer. In this procedure, the peptides were constructed on the resin beginning with the C-terminal protected amino acid. The resin support employed can be any suitable resin conventionally employed in the art for the solid phase preparation of polypeptides, preferably polystyrene which has been cross-linked with 0.5 to about 3 percent divinyl benzene, which has been either chloromethylated or hydroxymethylated to provide sites for ester formation with the initially introduced α-amino protected amino acid. For C-terminal amides, a dimethylbenzhydrylamine resin can be used. After completion of coupling of the sequence either the Boc protecting group was removed or left in place or it was removed and the N-terminal amino group acylated. Displacement of the protected fragment from the resin was accomplished using the appropriate amino alcohol.

An example of a hydroxymethyl resin is described by Bodanszky et al., Chem. Ind. (London) 38, 1597-98 (1966). A chloromethylated resin is commercially available from Bio Rad Laboratories, Richmond, California, and the preparation of such a resin is described by Stewart et ai., "Solid Phase Peptide Synthesis" (Freeman & Co., San Francisco 1969), Chapter 1, pp. 1-6. The protected amino acid can be bound to the resin by the procedure of Gisin, Helv. Chem.Acta, 56, 1476 (1973). Many resin bound, protected amino acids are commercially available. As an example, to prepare a polypeptide of this invention wherein the carboxy terminal end is a D-Glu residue, a tert-butyloxycarbonyl (Boc) protected D-Glu bound to a benzylated, hydroxymethylated phenylacetamidomethyl (PAM) resin can be used.

Following the coupling of the α-amino protected amino acid to the resin support, the protecting group is removed using any suitable procedure such as by using trifluoroacetic acid in methylene chloride, trifluoroacetic acid alone, or HCl in dioxane. The deprotection is carried out at a temperature of between 0°C and room temperature. Other standard cleaving reagents and conditions for removal of specific α-amino protecting groups may be used. After removal of the α-amino protecting group the other amino protected amino acids are coupled step-wise in the desired order. Alternatively, multiple amino acid groups may be coupled by the solution method prior to coupling with the resin supported amino acid sequence.

The α-amino protecting group employed with each amino acid introduced into the polypeptide sequence may be any such protecting group known to the art. Among the classes of α-amino protecting groups contemplated are (1) acyl type protecting groups such as: formyl, trifluoroacetyl, phthalyl, toluenesulfonyl (tosyl), benzenesulfonyl, nitrophenylsulfenyl, tritylsulfenyl, o-nitrophenoxyacetyl and α-chlorobutyryl; (2) aromatic urethane type protecting groups such as benzyloxycarbonyl and substituted benzyloxycarbonyl, such as p-chlorobenzyloxycarbonyl, p-nitrobenzylcarbonyl, p-bromobenzyloxycarbonyl, p-methoxybenzyloxycarbonyl, 1-(p-biphenylyl)-1-methylethoxycarbonyl, α,α-dimethyl-3,5-dimethoxybenzyloxycarbonyl and benzhydryloxycarbonyl; (3) aliphatic urethane type protecting groups such as tert--butyloxycarbonyl (Boc), diisopropylmethoxycarbonyl, isopropyloxycarbonyl, ethoxycarbonyl and allyloxycarbonyl; (4) cycloalkyl urethane type protecting groups such as cyclopentyloxycarbonyl, adamantyloxycarbonyl and cyclohexyloxycarbonyl; (5) thiourethane type protecting groups such as phenylthiocarbonyl; and (6) alkyl type protecting groups such as triphenylmethyl (trityl) and benzyl. The preferred α-amino protecting group is tert-butyloxycarbonyl.

As is known in the art of solid phase peptide synthesis many of the amino acids bear functionalities requiring protection during the chain preparation. The use and selection of the appropriate protecting group is within the ability of those skilled in the art and will depend upon the amino acid to be protected and the presence of other protected amino acid residues on the peptide. The selection of such a side chain protecting group is critical in that it must be one which is not removed by cleavage during cleavage of the protecting group of the α-amino moiety. For example, suitable side chain protecting groups for lysine are benzyloxycarbonyl and substituted benzyloxycarbonyl, said substituent being selected from halo (e.g., chloro, bromo, fluoro) and nitro (e.g., 2-chlorobenzyloxycarbonyl, p-nitrobenzyloxycarbonyl, 3,4-dichlorobenzyloxycarbonyl), tosyl, t-amyloxycarbonyl, t-butyloxycarbonyl and diisopropylmethoxycarbonyl. The alcoholic hydroxyl group of threonine and serine can be protected with an acetyl, benzoyl, tert-butyl, trityl, benzyl, 2,6-dichlorobenzyl or benzyloxycarbonyl group. The preferred protecting group is benzyl.

The selection of an appropriate coupling reagent is within the skill of the art. A particularly suitable coupling reagent where the amino acid to be added is Gln, Asn or Arg is N,N'-diisopropylcarbodiimide and 1-hydroxybenzotriazole. The use of these reagents prevents nitrile and lactam formation. Other coupling agents are (1) carbodiimides (e.g., N,N'-dicyclohexylcarbodiimide and N-ethyl-N'-(Υ-dimethylaminopropylcarbodiimide); (2) cyanamides (e.g., N,N-dibenzylcyanamide); (3) ketenimines; (4) isoxazolium salts (e.g., N-ethyl-5-phenyl-isoxazolium-3'-sulfonate; (5) monocyclic nitrogen containing heterocyclic amides of aromatic character containing one through four nitrogens in the ring such as imidazolides, pyrazolides, and 1,2,4-triazolides. Specific heterocyclic amides that are useful include N,N'-carbonyldiimidazole and N,N-carbonyl-di-1,2,4-triazole; (6) alkoxylated acetylene (e.g., ethoxyacetylene); (7) reagents which form a mixed anhydride with the carboxyl moiety of the amino acid (e.g., ethylchloroformate and isobutylchloroformate) or the symmetrical anhydride of the amino acid to be coupled (e.g., Boc-Ala-O-Ala-Boc), (8) nitrogen containing heterocyclic compounds having a hydroxy group on one ring nitrogen (e.g., N-hydroxyphthalimide, N-hydroxysuccinimide and 1-hydroxybenzotriazole) and (9) Castro's Reagent (BOP). Other activating reagents and their use in peptide coupling are described by Kapoor, J. Pharm. Sci., 59, pp. 1-27 (1970). Applicants prefer the use of the symmetrical anhydride as a coupling reagent for all amino acids except Arg, Asn and Gln.

Each protected amino acid or amino acid sequence is introduced into the solid phase reactor in about a four-fold excess and the coupling is carried out in a medium of dimethylformamide: methylene chloride (1:1) or in dimethylformamide alone or preferably methylene chloride alone. In cases where incomplete coupling occurs, the coupling procedure is repeated before removal of the α-amino protecting group, prior to the coupling of the next amino acid in the solid phase reactor. The success of the coupling reaction at each stage of the synthesis is monitored by the ninhydrin reaction as described by E. Kaiser et al, Analyt. Biochem. 34, 595 (1970).

Following the coupling of the α-amino protected amino acid to the resin support, the protecting group is removed using any suitable procedure such as by using trifluoroacetic acid in methylene chloride, trifluoroacetic acid alone, or HCl in dioxane. The deprotection is carried out at a temperature of between 0°C and room temperature. Other standard cleaving reagents and conditions for removal of specific α-amino protecting groups may be used. After removal of the α-amino protecting group the other amino protected amino acids are coupled step-wise in the desired order. Alternatively, multiple amino acid groups may be coupled by the solution method prior to coupling with the resin supported amino acid sequence.

After the desired amino acid sequence has been obtained, the peptide is removed from the resin and deprotected. This can be done by hydrolysis such as by treatment of the resin bound polypeptide with anhydrous liquid HF in the presence of scavengers (e.g. amisole). Typically protecting group removal is done after the peptide chain synthesis is complete but the protecting groups can be removed at any other appropriate time.

Purification and analysis of the deprotected peptide is accomplished by a number of standard procedures. The selection of the appropriate purification and analysis procedures is within the skill of the art. A suitable purification procedure is preparative HPLC. Analysis of the purified peptides can be done by analytical HPLC, amino acid analysis, fast atom bombardment mass spectrometry, and any other suitable means of analysis.

### Therapeutic Use:

The anticoagulant dose of an peptide derivative of this invention is from 0.2 mg/kg to 250 mg/kg of patient body weight per day depending on the patient, the severity of the thrombotic condition to be treated and the peptide derivative selected. The suitable dose for a particular patient can be readily determined. Preferably from 1 to 4 daily doses would be administered typically with from 5 mg to 100 mg of active compound per dose. The amount of a peptide of this invention required to inhibit or prevent blood coagulation in an extracorporeal medium such as stored whole blood can be readily determined by those skilled in the art.

Anticoagulant therapy is indicated for the treatment and prevention of a variety of thrombotic conditions, particularly coronary artery and cerebrovascular disease. Those experienced in this field are readily aware of the circumstances requiring anticoagulant therapy. The term "patient" used herein is taken to mean mammals such as primates, including humans, sheep, horses, cattle, pigs, dogs, cats, rats and mice. Inhibition of blood coagulation is useful not only in anticoagulant therapy of individuals having thrombotic conditions, but is useful whenever inhibition of blood coagulation is desirable, such as to prevent coagulation in stored whole blood and to prevent coagulation in other biological samples for testing or storage.

Although some of the peptide derivatives may survive passage through the gut following oral administration, applicants prefer non-oral administration, for example, subcutaneous, intravenous, intramuscular or intraperitoneal; administration by depot injection; by implant preparation; or by application to the mucous membranes, such as, that of the nose, throat and bronchial tubes, for example, in an aerosol can containing a peptide derivative of this invention in a spray or dry powder form.

For parenteral administration the compounds may be administered as injectable dosages of a solution or suspension of the compound in a physiologically acceptable diluent with a pharmaceutical carrier which can be a sterile liquid such as water and oils with or without the addition of a surfactant and other pharmaceutically acceptable adjuvants. Illustrative of oils which can be employed in these preparations are those of petroleum, animal, vegetable, or synthetic origin, for example, peanut oil, soybean oil, and mineral oil. In general, water, saline, aqueous dextrose and related sugar solutions, ethanol and glycols such as propylene glycol or polyethylene glycol are preferred liquid carriers, particularly for injectable solutions.

The compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic, silicone rubber manufactured by the Dow-Corning Corporation.

### EXAMPLES

This invention is illustrated by the following, nonlimiting examples.

### EXAMPLE 1

### Preparation of Anticoagulant Peptides.

The peptide was synthesized by solid-phase methods using 8.3 mmol of a 0.56 mmol/g Bod D-Glu(Bzl) Merrifield resin on a Vega semi-automated peptide synthesizer. Single symmetrical anhydride couplings were performed with 18.3 mmol Nα-Boc-amino acid (Peptides International), except in the case of Boc-Cha which required two coupling reactions to give a negative Kaiser test. Portions of the peptide resin (0.7 to 1.5g) were removed and set aside after cycles 3 through 8; for des-Glu⁵⁷ 1.36 g (0.50 mmol) of Boc-Pro-Ile-Pro-Glu(Bzl)-Glu(Bzl)-Ala-Cha-D-Glu(Bzl) Merrifield resin was reserved after cycle 8. The protected peptide fragment resin was transferred to an Applied Biosystems reaction vessel where Boc-Tyr(2-BrZ) was added using double symmetrical anhydride coupling with 2.0 mmol amino acid. The Nα-Boc protection was removed with 50% trifluoroacetic acid in methylene chloride, neutralized with three washings of 10% diisopropylethylamine in methylene chloride, washed three times with methylene chloride, and dried under nitrogen flow. The peptide was N∝ succinylated with double succinic anhydride coupling in dimethyl formamide, rinsed three times with dimethyl formamide, neutralized with two washes of 10% diisopropylethylamine in methylene chloride, washed three times with methylene chloride and dried *in_vacuo*. The peptide was deprotected and cleaved from the resin with anhydrous HF containing 5% anisol at 0°C for 60 min. The HF was removed in vacuo at 0°C; the peptide was extracted from the resin with 25% aqueous acetonitrile, frozen and lyophilized.

Preparative HPLC was performed on a C¹⁸ Beckman 50.8 x 150 mm column-with a 29.6-31.6% acetonitrile linear gradient over 15 min in 0.1% aqueous trifluoroacetic acid at 80 ml/min. The major peak (monitored at 280 nm) was collected and lyophilized yielding 281 mg of the desired product. Homogeneity was determined by HPLC: Vydac 218TP54 (4.6 x 250 mm) C¹⁸ column, 2.0 ml/min, t. = 1.5 min, time of elution with a 15-40% acetonitrile linear gradient over 25 min in 0.1% trifluoroacetic acid was 17.1 min.

Analysis of purified labeled peptides gave the desired molecular ion peak by FAB-MS and had an amino acid analysis in accordance with the desired peptide. In this way the following peptides have the stated physical properties specified below.

Samples were tested in a thrombin induced fibrin clot inhibition assay. All the solutions of the assay were made with an assay buffer containing 0.12 M sodium chloride, 0.01 M sodium phosphate, 0.01% sodium azide and 0.1% bovine serum albumin, pH 7.4. Bovine thrombin was titrated to an appropriate concentration so that fibrin clot formation could be monitored by a microtiter plate reader (Bio-Tek EL 309) within 60 min at 405 nm. This solution of thrombin (50 ul; 0.2 pmol) was added to the wells of a microtiter plate containing 50 ul of a solution of the synthetic peptide being tested. After 1 min agitation and an additional 10 mins incubation at 20°C, 100 ul of diluted plasma (1:10) in 0.1% EDTA was added and vortexed for 20 s. The turbidity of the solution was monitored by the autoreader at 5 min intervals. IC₅₀ is calculated from the results and is defined as the concentration of peptide which leads to half of the turbidity observed relative to a control containing no inhibitor. This is equivalent to a twofold increase in fibrin clot formation time. For the assays using human thrombin, it and bovine thrombin were titrated to concentrations which gave the fibrin clot at the same rate over a 30 min period. In this way the following peptides have the stated biological properties for the examples specified below where + signifies an IC₅₀ >25 uM and <200 µM, and ++ signifies an IC₅₀ < 25 µM.
1) Suc-Tyr-Pro-Ile-Pro-Glu-Glu-Ala-Cha-D-Glu-OH MW 1200 FAB-MS (MH)⁺ 1201

| | | | | |
|---|---|---|---|---|
| Z(3) | Pro(2) | Ile(1) | Tyr(1) | Ala(1) |
| 3.08 | 1.97 | 0.96 | 0.99 | 0.99 |

In vitro potency: +
2) H-Gly-Asp-Phe-Glu-Glu-Ile-Glu-Glu-Tyr-Leu-Gln-OH MW 1370 FAB-MS (MH)⁺ 1371

| | | | | | | |
|---|---|---|---|---|---|---|
| Z(5) | Ile(1) | Tyr(1) | Gly(1) | B(1) | Phe(1) | Leu(1) |
| 4.93 | 0.85 | 0.95 | 1.09 | 1.10 | 1.08 | 0.99 |

In vitro potency: +
3) Suc-Tyr-Glu-Pro-Tiq-Glu-Glu-Ala-Cha-D-Glu-OH MW 1278 FAB-MS (MH)⁺ 1279

| | | | | | |
|---|---|---|---|---|---|
| Z(4) | Pro(1) | Ile(1) | Tyr(1) | Ala(1) | Cha(1) |
| 4.05 | 0.93 | 0.96 | 0.95 | 1.03 | 1.03 |

In vitro potency: ++
4) Suc-Tyr-Glu-Pro-Ile-Pro-Glu-Glu-Ala-Gln-NH2 MW 1158 FAB-MS (MH)⁺ 1175

| | | | | |
|---|---|---|---|---|
| Z(4) | Pro(2) | Ile(1) | Tyr(1) | Ala(1) |
| 4.15 | 1.94 | 0.96 | 1.01 | 0.95 |

In vitro potency: ++
5) Suc-Tyr-Glu-Pro-Ile-Pro-Glu-Glu-Ala-Asn-OH MW 1161 FAB-MS (MH)⁺ 1162

| | | | | | |
|---|---|---|---|---|---|
| Z(3) | Pro(2) | Ile(1) | Tyr(1) | Ala(1) | B(1) |
| 3.14 | 1.95 | 0.95 | 1.00 | 0.96 | 1.01 |

In vitro potency: ++
6) Mal-Tyr-Pro-Ile-Pro-Glu-Glu-Ala-Cha-D-Glu-OH MW 1198 FAB-MS (MH)⁺ 1198

| | | | | |
|---|---|---|---|---|
| Z(3) | Pro(2) | Ile(1) | Tyr(1) | Ala(1) |
| 3.06 | 2.01 | 0.95 | 0.99 | 1.01 |

In vitro potency: +
7) Suc-Nap-Pro-Ile-Pro-Glu-Glu-Ala-Cha-D-Glu-OH MW 1234 FAB-MS (MH)⁺ 1234

| | | | |
|---|---|---|---|
| Z(3) | Pro(2) | Ile(1) | Ala(1) |
| 3.08 | 2.00 | 0.94 | 0.97 |

In vitro potency: ++
8) Suc-Tyr-Glu-Ile-Pro-Glu-Glu-Ala-Cha-D-Glu-OH MW 1232 FAB-MS (MH)⁺ 1233

| | | | | |
|---|---|---|---|---|
| Z(4) | Pro(1) | Ile(1) | Tyr(1) | Ala(1) |
| 4.07 | 0.99 | 0.96 | 0.98 | 0.99 |

In vitro potency: +
9) Suc-Tyr-Glu-Pro-Pro-Glu-Glu-Ala-Cha-D-Glu-OH MW 1216 FAB-MS (MH)⁺ 1217

| | | | |
|---|---|---|---|
| Z(4) | Pro(2) | Tyr(1) | Ala(1) |
| 4.05 | 1.97 | 0.98 | 1.00 |

In vitro potency: +
10) Suc-Tyr-Glu-Pro-Ile-Glu-Glu-Ala-Cha-D-Glu-OH MW 1232 FAB-MS (MH)⁺ 1233

| | | | | |
|---|---|---|---|---|
| Z(4) | Pro(1) | Ile(1) | Tyr(1) | Ala(1) |
| 4.10 | 1.01 | 0.94 | 0.96 | 0.99 |

In vitro potency: ++
11) Suc-Tyr-Glu-Pro-Ile-Pro-Glu-Glu-Ala-D-Glu-OH MW 1176 FAB-MS (MH)⁺ 1177

| | | | | |
|---|---|---|---|---|
| Z(4) | Pro(2) | Ile(1) | Tyr(1) | Ala(1) |
| 4.08 | 1.97 | 0.96 | 0.99 | 1.00 |

In vitro potency: ++
12) Suc-Tyr-Glu-Pro-Ile-Pro-Glu-Glu-Ala-Gln-OH MW 1175 FAB-MS (MH)⁺ 1175

| | | | | |
|---|---|---|---|---|
| Z(4) | Pro(2) | Ile(1) | Tyr(1) | Ala(1) |
| 4.11 | 1.98 | 0.93 | 1.01 | 0.97 |

In vitro potency: ++
13) Suc-Phe-Glu-Pro-Ile-Pro-Glu-Glu-Ala-Gln-NH2
MW 1158 FAB-MS (MH)⁺ 1159

| | | | | |
|---|---|---|---|---|
| Z(4) | Pro(2) | Ile(1) | Ala(1) | Phe(1) |
| 4.10 | 2.05 | 0.93 | 0.96 | 0.97 |

In vitro potency: +
14) Suc-Glu-Pro-Ile-Pro-Glu-Glu-Ala-Cha-D-Glu-OH
MW 1166 FAB-MS (MH)⁺ 1167

| | | | |
|---|---|---|---|
| Glx(4) | Pro(2) | Ile(1) | Ala(1) |
| 4.12 | 1.95 | 0.96 | 0.98 |

In vitro potency: +

## Claims

1. A peptide derivative selected from 
Suc-Tyr-Pro-Ile-Pro-Glu-Glu-Ala-Cha-D-Glu-OH;
H-Gly-Asp-Phe-Glu-Glu-Ile-Glu-Glu-Tyr-Leu-Gln-OH;
Suc-Tyr-Glu-Pro-Tiq-Glu-Glu-Ala-Cha-D-Glu-OH;
Suc-Tyr-Glu-Pro-Ile-Pro-Glu-Glu-Ala-Gln-NH2;
Suc-Tyr-Glu-Pro-Ile-Pro-Glu-Glu-Ala-Asn-OH;
Mal-Tyr-Pro-Ile-Pro-Glu-Glu-Ala-Cha-D-Glu-OH;
Suc-Nap-Pro-Ile-Pro-Glu-Glu-Ala-Cha-D-Glu-OH;
Suc-Tyr-Glu-Ile-Pro-Glu-Glu-Ala-Cha-D-Glu-OH;
Suc-Tyr-Glu-Pro-Pro-Glu-Glu-Ala-Cha-D-Glu-OH;
Suc-Tyr-Glu-Pro-Ile-Glu-Glu-Ala-Cha-D-Glu-OH;
Suc-Tyr-Glu-Pro-Ile-Pro-Glu-Glu-Ala-D-Glu-OH;
Suc-Tyr-Glu-Pro-Ile-Pro-Glu-Glu-Ala-Gln-OH;
Suc-Phe-Glu-Pro-Ile-Pro-Glu-Glu-Ala-Gln-NH2;
Suc-Glu-Pro-Ile-Pro-Glu-Glu-Ala-Cha-D-Glu-OH;
 or a pharmaceutically acceptable salt thereof.

2. A process for preparing a peptide derivative according to claim 1 or a pharmaceutically acceptable salt thereof comprising the steps of
a) using a resin with a suitably bound C-terminal protected amino acid;
b) sequentially coupling the other alpha amino protected amino acids to achieve the protected amino acid sequence claimed; and
c) removing said protecting groups and purifying the desired peptide.

3. A peptide derivative or a pharmaceutically acceptable salt thereof according to claim 1 or a mixture thereof for use as a pharmaceutically active substance.

4. A peptide derivative or a pharmaceutically acceptable salt thereof according to claim 1 or a mixture thereof for use as an anticoagulant.

5. Use of the peptide derivatives of claim 1 for the preparation of a pharmaceutical composition.

6. Use according to claim 5 wherein the composition is useful as an anticoagulant.

7. A pharmaceutical composition containing a peptide derivative as defined in claim 1 and optionally a pharmaceutically acceptable carrier, diluent and/or excipient.

8. The composition of claim 7 for reducing blood coagulation.

9. A method of reducing blood coagulation in an extracorporal medium which comprises contacting the medium with a blood anti-coagulation effective amount of a peptide of claim 1.

10. A method for inhibiting blood coagulation in an extracorporal medium by contacting a peptide of claim 1 with extracorporal medium.

## Patentansprüche

1. Peptidderivat, ausgewählt aus 
Suc-Tyr-Pro-Ile-Pro-Glu-Glu-Ala-Cha-D-Glu-OH;
H-Gly-Asp-Phe-Glu-Glu-Ile-Glu-Glu-Tyr-Leu-Gln-OH;
Suc-Tyr-Glu-Pro-Tiq-Glu-Glu-Ala-Cha-D-Glu-OH;
Suc-Tyr-Glu-Pro-Ile-Pro-Glu-Glu-Ala-Gln-NH2;
Suc-Tyr-Glu-Pro-Ile-Pro-Glu-Glu-Ala-Asn-OH;
Mal-Tyr-Pro-Ile-Pro-Glu-Glu-Ala-Cha-D-Glu-OH;
Suc-Nap-Pro-Ile-Pro-Glu-Glu-Ala-Cha-D-Glu-OH;
Suc-Tyr-Glu-Ile-Pro-Glu-Glu-Ala-Cha-D-Glu-OH;
Suc-Tyr-Glu-Pro-Pro-Glu-Glu-Ala-Cha-D-Glu-OH;
Suc-Tyr-Glu-Pro-Ile-Glu-Glu-Ala-Cha-D-Glu-OH;
Suc-Tyr-Glu-Pro-Ile-Pro-Glu-Glu-Ala-D-Glu-OH;
Suc-Tyr-Glu-Pro-Ile-Pro-Glu-Glu-Ala-Gln-OH;
Suc-Phe-Glu-Pro-Ile-Pro-Glu-Glu-Ala-Gln-NH2;
Suc-Glu-Pro-Ile-Pro-Glu-Glu-Ala-Cha-D-Glu-OH;
 oder ein pharmazeutisch verträgliches Salz davon.

2. Verfahren zur Herstellung eines Peptidderivats oder eines pharmazeutisch verträglichen Salzes davon gemäß Anspruch 1, umfassend die Schritte
a) Verwendung eines Harzes mit einer geeignet gebundenen, C-terminal geschützten Aminosäure;
b) aufeinanderfolgendes Verknüpfen der anderen α-Aminogeschützten Aminosäuren, um die beanspruchte geschützte Aminosäuresequenz herzustellen; und
c) Entfernen der Schutzgruppen und Reinigen des gewünschten Peptids.

3. Peptidderivat oder ein pharmazeutisch verträgliches Salz davon gemäß Anspruch 1 oder ein Gemisch davon zur Verwendung als pharmazeutischer Wirkstoff.

4. Peptidderivat oder ein pharmazeutisch verträgliches Salz davon gemäß Anspruch 1 oder ein Gemisch davon zur Verwendung als Antikoagulans.

5. Verwendung der Peptidderivate von Anspruch 1 zur Herstellung eines Arzneimittels.

6. Verwendung gemäß Anspruch 5, wobei das Arzneimittel als Antikoagulans verwendbar ist.

7. Arzneimittel, enthaltend ein wie in Anspruch 1 definiertes Peptidderivat und gegebenenfalls einen pharmazeutisch verträglichen Träger, ein Verdünnungsmittel und/oder ein Exzipiens.

8. Arzneimittel von Anspruch 7 zur Verminderung der Blutkoagulation.

9. Verfahren zur Verminderung der Blutkoagulation in einem extrakorporalen Medium, das das Inkontaktbringen des Mediums mit einer blutantikoagulierend wirkenden Menge eines Peptids von Anspruch 1 umfaßt.

10. Verfahren zur Hemmung der Blutkoagulation in einem extrakorporalen Medium durch Inkontaktbringen eines Peptids von Anspruch 1 mit dem extrakorporalen Medium.

## Revendications

1. Dérivé peptide choisi parmi 
Suc-Tyr-Pro-Ile-Pro-Glu-Glu-Ala-Cha-D-Glu-OH;
H-Gly-Asp-Phe-Glu-Glu-Ile-Glu-Glu-Tyr-Leu-Gln-OH;
Suc-Tyr-Glu-Pro-Tiq-Glu-Glu-Ala-Cha-D-Glu-OH;
Suc-Tyr-Glu-Pro-Ile-Pro-Glu-Glu-Ala-Gln-NH2;
Suc-Tyr-Glu-Pro-Ile-Pro-Glu-Glu-Ala-Asn-OH;
Mal-Tyr-Pro-Ile-Pro-Glu-Glu-Ala-Cha-D-Glu-OH;
Suc-Nap-Pro-Ile-Pro-Glu-Glu-Ala-Cha-D-Glu-OH;
Suc-Tyr-Glu-Ile-Pro-Glu-Glu-Ala-Cha-D-Glu-OH;
Suc-Tyr-Glu-Pro-Pro-Glu-Glu-Ala-Cha-D-Glu-OH;
Suc-Tyr-Glu-Pro-Ile-Glu-Glu-Ala-Cha-D-Glu-OH;
Suc-Tyr-Glu-Pro-Ile-Pro-Glu-Glu-Ala-D-Glu-OH;
Suc-Tyr-Glu-Pro-Ile-Pro-Glu-Glu-Ala-Gln-OH;
Suc-Phe-Glu-Pro-Ile-Pro-Glu-Glu-Ala-Gln-NH2;
Suc-Glu-Pro-Ile-Pro-Glu-Glu-Ala-Cha-D-Glu-OH;
 ou un sel acceptable pharmaceutiquement de celui-ci.

2. Procédé de préparation d'un dérivé peptide selon la revendication 1 ou un sel acceptable pharmaceutiquement de celui-ci comprenant les étapes de
a) l'utilisation d'une résine avec un acide aminé protégé en C-terminal convenablement lié;
b) le couplage séquentiel des autres acides aminés protégés en alpha amino pour obtenir la séquence d'acides aminés protégés revendiquée; et
c) l'enlèvement desdits groupes protecteurs et la purification du peptide désiré.

3. Dérivé peptide ou un sel acceptable pharmaceutiquement de celui-ci selon la revendication 1 ou un mélange de celui-ci pour l'utilisation comme substance active pharmaceutiquement.

4. Dérivé peptide ou un sel acceptable pharmaceutiquement de celui-ci selon la revendication 1 ou un mélange de celui-ci pour l'utilisation comme anticoagulant.

5. Utilisation des dérivés peptides selon la revendication 1 pour la préparation d'une composition pharmaceutique.

6. Utilisation selon la revendication 5, caractérisée en ce que la composition est utile comme anticoagulant.

7. Composition pharmaceutique contenant un dérivé peptide tel que défini dans la revendication 1 et éventuellement un support, un diluant et/ou un excipient acceptables pharmaceutiquement.

8. Composition selon la revendication 7 pour réduire la coagulation sanguine.

9. Procédé de réduction de la coagulation sanguine dans un milieu extracorporel qui comprend la mise en contact du milieu avec une quantité efficace d'anticoagulation d'un peptide selon la revendication 1.

10. Procédé d'inhibition de la coagulation sanguine dans un milieu extracorporel par mise en contact d'un peptide selon la revendication 1 avec le milieu extracorporel.
